# EUROPEAN PATENT APPLICATION

(11) **EP 3 311 852 A1**
(43) Date of publication of application: **25.04.2018**
(21) Application number: 17382706.4
(22) Date of filing: 24.10.2017
(51) Int. Cl.: A61L 12/08, C11D 3/00, B41M 1/04

(54) **OPHTHALMOLOGICAL PRODUCT, PRODUCTION METHOD AND CONTACT LENS CLEANING AND DISINFECTION METHOD**

(30) Priority: 24.10.2016 ES 201631361
(71) Applicant: DISOP.S.A., 28108 Alcobendas (Madrid) (ES)
(72) Inventor: HERRERO COLMENERO, Raquel, 28108 ALCOBENDAS (Madrid) (ES); CIAURRIZ GORTARI, Paula, 28108 ALCOBENDAS (Madrid) (ES); CABRERA ASENSIO, Aarón, 28108 ALCOBENDAS (Madrid) (ES)
(74) Representative: Herrero & Asociados, S.L.

(57) **Abstract**

The present invention relates to an ophthalmological product comprising a concentration of silver ions, which are released from a polymer insert, that is very low but effective in cleaning and disinfection, as well as to the production method for producing same. The present invention also relates to a method for cleaning and disinfecting contact lenses with the product of the invention.

## Description

The present invention relates to an ophthalmological product comprising a concentration of silver ions that is very low but effective in cleaning and disinfection, as well as to the production method for producing same. The present invention also relates to a method for cleaning and disinfecting contact lenses with the product of the invention.

### Background of the Invention

Disinfecting contact lenses with ophthalmological products containing silver ions, such as the composition described in international patent application WO2005088351 is known, where said application describes an insert which is placed inside a case containing a fluid. When the insert is covered by the fluid, the silver nanoparticles and/or a titanium oxide photocatalyst are released into the fluid to react with the bacteria and with the contact surface for cleaning and disinfecting them. The concentration of the material must be above 0.001%, and it indicates that higher concentrations are preferred because the higher the concentration of silver particles is, the faster the contact lens cleaning will be. Silica oxide or titanium oxide is used in the insert as support for the silver nanoparticles.

Patent application US5320843 describes a solution with antibacterial effect comprising an element molded from a plastic resin including an inorganic carrier retaining antibacterial metal ions, where the plastic resin element and the solution are in contact. In a particular case, the ion carrier is zeolite, and the metal ions are silver ions incorporated in the zeolite at a percentage by weight of 0.001% to 10%, based on the weight of anhydrous zeolite.

Based on what is known in the state of the art, the development of new ophthalmological products is of great interest.

### Description of the Invention

The solution of the invention solves different problems of the state of the art, such as the production of a very low concentration of silver ions that is effective and does not have the contraindications of higher concentrations, such as toxicity.

The ophthalmological product of the present invention comprises a solution with a polymer insert therein. One of the faces of the polymer insert is printed with silver nanoparticle ink, the silver nanoparticles of which are released into the solution. The silver ion release mechanism takes place on the surface of the insert covered with metallic silver nanoparticles in the form of a layer, from the moment of filling the case. From that moment on, the ion diffusion-reaction process is triggered, until a maximum equilibrium concentration is achieved.

The inventors have determined that the solution is not more effective with higher concentrations than those described in the present invention, probably due to the occurrence of oxidation-reduction reactions which convert silver ions into charge-free silver that has no activity. As shown below in Table 3, when the concentration of silver is twice the concentration within the range of the invention, better disinfecting effects are not achieved.

Therefore the first aspect of the invention relates to an ophthalmological product comprising a solution for cleaning and disinfection comprising Ag⁺ ions at a concentration comprised between 0.000010% and 0.000050% expressed in w/V, which ions are released from a polymer insert printed with silver nanoparticle ink, immersed in the solution.

The printing of the silver nanoparticles is performed by means of the flexography technique. Flexography allows printing patterns defined by means of an embossed photopolymer stamp which is contacted with an inking roller. The inking roller works to apply the ink uniformly on the entire, embossed stamp surface. Each insert therefore receives a precise and uniform amount of ink in each printing. The ink contains the silver nanoparticles which will then release Ag⁺ ions into the solution.

Therefore, a second aspect of the invention relates to a production method for producing the ophthalmological product of the invention, comprising the steps of:
a) web printing a polymer insert with an inking concentration comprised between 15 g/m² - 25 g/m² by means of flexography;
b) curing the print at a temperature comprised between 130°C - 180°C and for a time comprised between 60 s to 120 s;
c) introducing the polymer insert printed with silver ink formed by silver nanoparticles into a container;
d) filling the container under aseptic conditions with a contact lens cleaning and disinfecting solution;
e) allowing the insert to sit in the solution until the concentration of Ag⁺ ions is comprised between 0.000010% and 0.000050% expressed in w/V.

The third aspect of the invention relates to a contact lens cleaning and disinfection method comprising the steps of:
a) adding the solution comprised in the ophthalmological product of the invention to the contact lens;
b) keeping the lens in the solution of the ophthalmological product of the invention for the time required for cleaning and disinfecting same.

### Brief Description of the Drawings

To better understand what has been set forth, several drawings in which a practical embodiment is depicted in a merely schematic and non-limiting manner are attached.

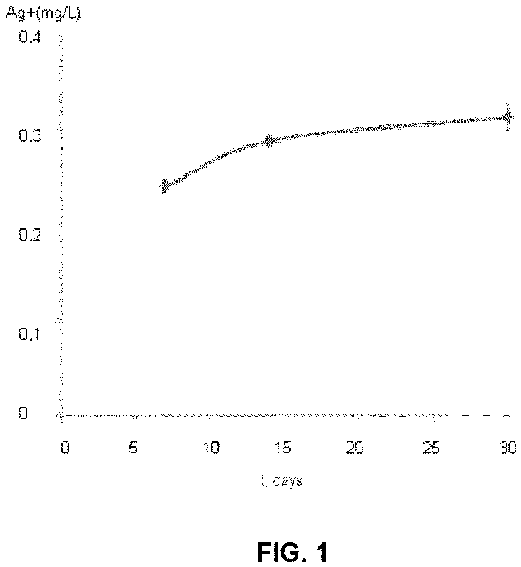

Figure 1 depicts the concentration of Ag ions in mg/L over time in days.

### Description of a Preferred Embodiment

As mentioned, the first aspect of the invention relates to an ophthalmological product comprising a contact lens cleaning and disinfecting solution comprising Ag⁺ ions at a concentration comprised between 0.000010% and 0.000050% expressed in w/V, which ions are released from a polymer insert printed with silver nanoparticle ink, immersed in the solution.

Preferably, in addition to Ag⁺ ions, the contact lens cleaning and disinfecting solution comprises sodium chloride in a range comprised between 0.47% and 0.79% expressed in w/V, disodium phosphate in a range comprised between 0.03% and 0.90% expressed in w/V, sodium phosphate in a range comprised between 0.005% and 0.15% expressed in w/V, sodium citrate in a range comprised between 0.01% and 0.30% expressed in w/V, hyaluronic acid in a range comprised between 0.003% and 0.10% expressed in w/V, poloxamer in a range comprised between 0.01% and 0.30% expressed in w/V.

Preferably, the concentration of Ag⁺ is comprised between 0.000020% and 0.000040% expressed in w/V. More preferably, the concentration of Ag⁺ is comprised between 0.000025% and 0.000035% expressed in w/V.

The polymer insert is preferably made of a polymer material which withstands temperatures up to 180°C without deforming. Particularly, the polymer is PET.

Preferably, the insert has a V shape which allows the positioning thereof in the container through the opening, in addition to offering a sufficient surface for releasing ions into the medium. Furthermore, the design is optimized to make better use of the surface to be printed and of the progress of the printing roller. Particularly, each of the insert rectangles measured 13 x 120 mm.

Preferably, the surface area of the insert coated with silver nanoparticles with respect to the volume of the solution is comprised in a range between 33 cm²/L and 50 cm²/L. Preferably, printing is performed with ink having a 40% silver nanoparticle content and the nanoparticle size is about 50 nm.

As mentioned, the second aspect of the invention relates to a production method for producing ophthalmological product of the invention comprising the steps of:
a) web printing a polymer insert with an inking concentration comprised between 15 g/m² - 25 g/m² by means of flexography;
b) curing the print at a temperature comprised between 130°C-180°C and for a time comprised between 60 s to 120 s;
c) introducing the polymer insert printed with silver ink formed by silver nanoparticles into a container;
d) filling the container under aseptic conditions with a contact lens disinfection and cleaning solution;
e) allowing the insert to sit in the solution until the concentration of Ag⁺ ions is comprised between 0.000010% and 0.000050% expressed in w/V.

Preferably, the step of curing the ink is performed in a tunnel at a temperature between 130°C and 150°C for a time comprised between 60 s and 120 s.

Preferably, the time of step e) is comprised between five and fifteen days.

Preferably, the ink viscosity is less than 100 cP. Preferably, the silver content of the ink is between 30% and 60%. More preferably, the silver content of the ink is between 35% and 45%.

Preferably, the cleaning solution of step d) comprises: sodium chloride in a range comprised between 0.47% and 0.79% expressed in w/V, disodium phosphate in a range comprised between 0.03% and 0.90% expressed in w/V, sodium phosphate in a range comprised between 0.005% and 0.15% expressed in w/V, sodium citrate in a range comprised between 0.01% and 0.30% expressed in w/V, hyaluronic acid in a range comprised between 0.003% and 0.10% expressed in w/V, poloxamer in a range comprised between 0.01% and 0.30% expressed in w/V.

The contact lens cleaning and disinfection method comprising the steps of:
a) adding the solution comprised in the ophthalmological product defined in the invention to the contact lens and
b) keeping the lens in the solution of the ophthalmological product of the invention for the time required for cleaning and disinfecting same. The time of this step b) is at least four hours.

### Example of the Invention

In the present example, the polymer insert is an FDA (Food & Drug Administration) food-grade PET (polyethylene terephthalate) insert having dimensions of 13 x 120 mm (1560 mm²).

A V-shaped insert printed on one face was introduced in each of the cases. The inserts were printed on one face with silver nanoparticle-based ink by means of PET web flexographic printing. The manufacture and filling of the case was performed under aseptic conditions, i.e., by sterilizing filtration. The packaging materials (container, cap and insert were first sterilized by means of the technique of applying beta irradiation above 25 kGy. Finally, the packaged product was subjected to a sterility test in accordance with the European Pharmacopeia.

In the present example, the solution has the following composition shown in Table 1.

**Table 1. Composition of the contact lens cleaning and disinfecting solution**

| Ingredient | %(w/V) |
|---|---|
| Sodium chloride | 0.63 |
| Disodium phosphate 2H₂O | 0.30 |
| Sodium phosphate 2H₂O | 0.05 |
| Sodium citrate | 0.10 |
| Poloxamer | 0.10 |
| Hyaluronic acid | 0.0175 |
| Ag⁺ | 0.000034 |
| Purified water | q.s. |

Tests were performed according to the ISO 14729 standard in order to know the disinfecting capacity of the composition.

**Table 2. Results of the test according to ISO 14729 standard**

| Microorganism | t=3h | t=8h | t=24h |
|---|---|---|---|
| *P. aeruginosa* | 3.55 | 4.69 | 5.89 |
| *S. aureus* | 3.05 | 4.55 | 5.83 |
| *S. marcescens* | 3.54 | 5.57 | 5.57 |
| *C. albicans* | **0.89** | 1.46 | 1.64 |
| *F. solani* | 1.63 | 2.93 | 5.23 |

Except for *C*. *albicans,* the rest complies with the test requirements of a 3-log reduction in the number of cells for bacteria and 1-log reduction for fungi after 3 hours. The tests were performed in triplicate. It was confirmed that the objectives are not improved with more silver. Table 3 shows the results for the cases where two inserts that are printed with silver nanoparticles and have the same dimensions of 13 x 120 mm were introduced. Oxidation-reduction reactions in which Ag⁺ ions are turned into metal silver Ag⁰ that has no activity are likely to commence with more silver and so better results are not obtained with two inserts instead of one.

**Table 3. Results of the test in which the concentration of silver is twice the concentration defined in the composition of Table 1, the rest of the components being kept the same**

| Microorganism | t=3h | t=8h | t=24h |
|---|---|---|---|
| *P. aeruginosa* | **N.D.(*)** | 4.94 | 5.89 |
| *S. aureus* | 3.23 | 4.06 | 5.83 |
| *S. marcescens* | 4.04 | 4.67 | 4.72 |
| *C. albicans* | **0.41** | **0.94** | **1.12** |
| *F. solani* | **1.12** | **2.05** | 5.23 |

| | | | |
|---|---|---|---|
| (*) N.D. Not determined | | | |

An ion release test was performed. The results of this test are shown in Figure 1, the release of silver takes place particularly in the first 7 days, increasing subsequently until day 15 and remaining virtually the same thereafter, until reaching free silver levels of about 0.30 mg/L.

Preservative efficacy testing was also performed. The preparation also passed the test of the ISO 14730 standard - Ophthalmic optics. Contact lens care products. Antimicrobial preservative efficacy testing and guidance on determining discard date once the case is opened (ISO 14730:2014).

**Table 4. Results of the preservative capacity**

| Microorganis m | t= 0 | t=14 days; 25%B R | t=23 days; 25%B R | t=23 days; 25%A R | t=44 days; 50%B R | t=44 days; 50%A R | t=68 days; 75%B R | t=68 days; 75%A R | t=92 day s; 100 % |
|---|---|---|---|---|---|---|---|---|---|
| *Candida albicans* | 1, 6 10 ⁶ | <10 | <10 | 4.4 10³ | <10 | 7.7 10² | <10 | 1.4 10³ | <10 |
| *Aspergillus brasiliensis* | 1, 1 10 ⁶ | 9.0 10⁴ | 2.0 10⁴ | 2.7 10³ | 5.0 10² | 4.0 10³ | 2.0 10 | 1.3 10³ | 2.0 10 |
| *Escherichia coli* | 9, 4 10 ⁵ | <10 | <10 | 3.0 10³ | <10 | 3.7 10³ | <10 | 1.1 10³ | <10 |
| *Pseudomon* as *aeruginosa* | 8, 9 10 ⁵ | <10 | <10 | 3.4 10³ | <10 | 1.1 10³ | <10 | 1.1 10³ | <10 |
| *Staphylococ cus aureus* | 1, 0 10 ⁶ | <10 | <10 | 2.3 10³ | <10 | 1.7 10³ | <10 | 1.6 10³ | <10 |

The results demonstrate that the formulation of the example contains an amount of Ag⁺ sufficient to reduce the microbial load of 5 microorganisms for 92 days and after successive re-inoculations.

These results demonstrate that the formulation can be used as a base for ophthalmic solutions (moisturizing drops, lens maintenance products, eye baths, etc.)

Biological safety testing was also performed where it was confirmed that the ophthalmological product of the invention complies with the biological safety requirements of ISO 10993-1(2009) for invasive medical devices that are in permanent contact with the mucous membrane.

| **Tests** | **Results** |
|---|---|
| Systemic subchronic toxicity test | The product does not show any toxicity after the intraperitoneal administration of repeated doses in rodents for 90 days |
| Ames test | The product is not carcinogenic because it does not have any mutagenic capacity at the tested concentrations |
| Cytotoxicity | There are no significant differences between the test of the product and the negative control, indicating that the product is not cytotoxic. |
| Eye irritation | The primary irritation index has a value of 0, meaning that it is classified as non-irritant. |
| Sensitization test | The product is not a sensitizer. |

## Claims

1. Ophthalmological product comprising a solution for cleaning and disinfection, **characterized in that** it comprises Ag⁺ ions at a concentration comprised between 0.000010% and 0.000050% expressed in w/V, which ions are released from a polymer insert printed with silver nanoparticle ink, immersed in the solution.

2. Ophthalmological product according to claim 1, **characterized in that** the solution further comprises: sodium chloride in a range comprised between 0.47% and 0.79% expressed in w/V, disodium phosphate in a range comprised between 0.03% and 0.90% expressed in w/V, sodium phosphate in a range comprised between 0.005% and 0.15% expressed in w/V, sodium citrate in a range comprised between 0.01% and 0.30% expressed in w/V, hyaluronic acid in a range comprised between 0.003% and 0.10% expressed in w/V, poloxamer in a range comprised between 0.01% and 0.30% expressed in w/V.

3. Ophthalmological product according to any of claims 1 to 2, **characterized in that** the polymer forming the polymer insert is PET.

4. Production method for producing the ophthalmological product according to claim 1, comprising the steps of:
a) web printing a polymer insert with an inking concentration comprised between 15 g/m² - 25 g/m² by means of flexography;
b) curing the print at a temperature comprised between 130°C - 180°C and for a time comprised between 60 s to 120 s;
c) introducing the polymer insert printed with silver ink formed by silver nanoparticles into a container;
d) filling the container under aseptic conditions with a contact lens disinfection and cleaning solution;
e) allowing the insert to sit in the solution until the concentration of Ag⁺ ions is comprised between 0.000010% and 0.000050% expressed in w/V.

5. Method according to claim 4, **characterized in that** the sitting time of step e) is comprised between five and fifteen days.

6. Contact lens cleaning and disinfection method comprising the steps of:
a) adding the solution comprised in the ophthalmological product defined in any of claims 1 to 3 to the contact lens
b) keeping the contact lens in the solution of the ophthalmological product defined in any of claims 1 to 3 for the time required for cleaning and disinfecting same.

7. Method for cleaning according to claim 6, where the time of step b) is at least four hours.
